# EUROPÄISCHE PATENTSCHRIFT

(11) **EP 0 849 715 B1**
(45) Veröffentlichungstag und Bekanntmachung des Hinweises auf die Patenterteilung: **25.09.2002**
(21) Anmeldenummer: 97120923.4
(22) Anmeldetag: 28.11.1997
(51) Int. Cl.: G08B 21/00, A61B 5/11

(54) **Verfahren und Anordnung zur Erfassung von Sturzsituationen gesundheitsgefährdeter Personen**
Method and apparatus for detecting the fall of a sick person
Procédé et dispositif pour détecter la chute d'une personne malade

(30) Priorität: 21.12.1996 DE 19653773
(43) Veröffentlichungstag der Anmeldung: 24.06.1998
(73) Patentinhaber: GGT Deutsche Gesellschaft für Gerontotechnik mbH, 58638 Iserlohn (DE)
(72) Erfinder: Jellinghaus, Rolf, 58644 Iserlohn (DE); Jentsch, Gerhard, 58636 Iserlohn (DE); Joska, Rolf, 46119 Oberhausen (DE); Kowalski, Friedhelm, 58638 Iserlohn (DE); Langbein, Peter, 58708 Menden (DE); Schlieck, Gerhard, 58566 Kierspe (DE)

(56) Entgegenhaltungen:
- WO-A-88/07350
- DE-A- 2 532 504
- DE-A- 3 004 411
- US-A- 4 829 285
- US-A- 4 858 622
- US-A- 5 008 654
- US-A- 5 317 305
- US-A- 5 402 107

## Beschreibung

Die Erfindung betrifft ein Verfahren und eine Anordnung zur Durchführung des Verfahrens zur Erfassung von Sturzsituationen gesundheitsgefährdeter Personen, insbesondere Kranken, alten Menschen und Personen gefährdeter Berufsgruppen, wobei mittels eines am Körper der zu überwachenden Person zu tragenden Überwachungsgerätes Informationen von zeitlichen Bewegungsabläufen und Bewegungszuständen vor, während sowie nach einer Sturzsituation einer gesundheitsgefährdeten Person erfaßt sowie ausgewertet werden und bei auftretenden Differenzen ein Notruf ausgelöst wird, wobei dieser Notruf in eine Notrufzentrale oder bei einer Bezugsperson ausgelöst werden kann.

Aus dem Stand der Technik sind für gefährdete Berufsgruppen, beispielsweise Wachdienste, Personal in Kraftwerken oder Waldarbeiter eine Vielzahl von Sicherheitssystemen zum Auffinden der oftmals allein arbeitenden Personen bekannt, indem eine gesundheitlich kritische Situation mittels einer am Körper der zu überwachenden Person zu tragenden Überwachungseinheit ermittelt und über Funksignale an eine Zentrale übermittelt wird. Als Kriterium zur Erkennung von kritischen Situationen der gefährdeten Person wird bei diesen Systemen insbesondere die Neigung bzw. die Lage der Person mittels eines Lage- oder Neigungssensors erfaßt und bewertet, oder es wird der Körperzustand der Bewegungslosigkeit einer Person durch einen Bewegungssensor erfaßt.

Aus der DE-U 94 08 119.0 ist ein Alarmsystem für gefährdete Personen bekannt, bei dem eine am Körper der gefährdeten Person zu tragende Überwachungseinheit bei Veränderung von lebenswichtigen Körperfunktionen ein Funksignal zur Standorterkennung der Person aussendet.
Aus der DE 30 04 411 A1 sind weiterhin ein Verfahren und eine Vorrichtung zur Sicherung gefährdeter Personen bekannt, wobei das von der Person zu tragende Hilfsgerät ein Signalgeber ist, der drahtlos automatisch und willensunabhängig einen Alarm auslöst, wenn er eine bestimmte Zeitdauer in einer definierten Lage verbleibt.

Zur Bestimmung von Lage- und Bewegungszuständen sind eine Vielzahl von Sensoren, die auf der Basis unterschiedlichster optischer und mechanischer Prinzipien arbeiten, bekannt. Beispielsweise ist aus der DE 42 37 953 C1 ein auf dem Prinzip der Libelle arbeitender Neigungssensor, aus der DE 41 14 992 C1 und der DE 42 36 328 C1 ein Neigungs- und Beschleunigungssensor, der eine schwerkraftabhängige Eigenverformung eines Kontaktkörpers aus einem Material, etwa dem für Neigungssensoren an sich bekannten Quecksilber, welches bei Annäherung an die Schwerelosigkeit von einer linsenförmigen in eine kugelförmige Gestalt übergeht, in der Weise ausnutzt, daß mittels eines einzigen Sensors eine kombinierte Lage- und Neigungsmessung durchgeführt wird und auch, wie in der DE 41 14 992 dargestellt, eine Horizontalbeschleunigung und in der DE 42 36 328 eine Vertikalbeschleunigung gemessen wird, wobei diese Sensoren zur Anzeige kritischer Fahrzustände in Kraftfahrzeugen eingesetzt werden.

Ein Neigungssensor, mit dem sowohl die Richtung, als auch der Betrag einer Neigung in einem Raster mit einer als Lageindikator dienenden Kugel erfaßt wird, ist in der DE 38 31 144 A1 beschrieben. Es lassen sich dadurch mehrere Raumwinkelbereiche abdecken, so daß damit entschieden werden kann, ob Lageabweichungen nicht über ein vorgegebenes Maß hinausgehen.

Eine weitere Art von Neigungssensoren, die optoelektronischen Neigungssensoren mit einem Strahlungssender und einem Strahlungsempfänger werden benutzt, um als Gleichgewichtsorgan die Auslenkung eines Systems aus einer Ruhelage zu erfassen.

Bei den bekannten technischen Lösungen zur Erfassung gefährdeter Personen in einem bestimmten Umgebungsbereich besteht bei den technischen Lösungen, bei denen die zu überwachenden Personen einen Sender am Körper tragen, ein Nachteil darin, daß einerseits zu viele funktechnische Kriterien berücksichtigt werden müssen oder daß andererseits damit die Funktion eines Herzschrittmachers beeinträchtigt werden kann. Außerdem kann es mit diesen Systemen zu einem Fehlalarm kommen, wenn beispielsweise die Bewegungslosigkeit einer Person auf einer gewollten sitzenden und ruhigen Körperhaltung oder einer starken Körpemeigung auf einer gewollten Liegeposition einer Person beruht. Anderseits kann bei diesen Systemen ein Alarm ausbleiben, wenn trotz eines Sturzes eine relativ aufrechte Körperhaltung besteht und die betreffende Person sich trotz der Verletzungen noch geringfügig bewegt, wie es bei einem Treppensturz der Fall sein kann.

Ausgehend von diesem Stand der Technik liegt der Erfindung die Aufgabe zugrunde, ein Verfahren und eine Anordnung zur Durchführung des Verfahrens zu schaffen, mit denen gesundheitskritische Zustände von Personen erkannt und ein willensabhängiger und/oder willensunabhängiger Notruf ausgelöst wird.

Zur Lösung dieser Aufgabe schlägt die Erfindung ein Verfahren vor, bei dem mittels eines, von einer gesundheitsgefährdeten Person an einem Oberkörper zu tragenden Überwachungsgerätes in vorgegebenen wählbaren Zeitintervallen sowohl lage- und bewegungsabhängige Meßwertfolgen von Neigungs- und Lageänderungen der Person als auch Meßwertfolgen über die Geschwindigkeit sowie die Beschleunigung von Bewegungsabläufen und den Aufprall der Person mittels eine in dem Überwachungsgerät vorgesehenen Neigungs- und Bewegungssensors erfaßt und mit Meßwertfolgen von bekannten vorgegebenen Bewegungsabläufen der Person verglichen werden, wobei bei Überschreitung maximaler Differenzwerte als Folge einer Sturzsituation der gesundheitsgefährdeten Person ein Voralarm sowie ein willensabhängiger und/oder willensunabhängiger Notruf ausgelöst werden.

Bevorzugt ist vorgesehen, daß das von der gesundheitsgefährdeten Person zu tragende Überwachungsgerät die Lage- und Neigungsänderungen dieser Person sowie deren zeitlichen Verlauf mittels eines in 2 Ebenen wirkenden Neigungs- und Bewegungssensors mißt, wobei die Meßgrößen, wie Geschwindigkeit, Beschleunigung, Neigung und Richtung erfaßt und die kritischen Neigungs- und Bewegungszustandsänderungen der gesundheitsgefährdeten Person als Muster in einem neuronalen Netz ermittelt sowie bewertet werden und wobei gleichzeitig vorliegende Informationen einzeln oder in einem funktionellen Zusammenhang zur Bewertung dienen und daraus nach Vergleich mit den in dem Überwachungsgerät gespeicherten Meßwertfolgen vorgegebener bekannter Bewegungsabläufe der Person bei auftretenden Differenzen sowie bei Überschreitung maximaler Differenzwerte Signale gebildet werden, die einen Voralarm auslösen, der bei Nichtlöschung einen Notruf auslöst.

Vorteilhaft ist es, daß die Änderung der Amplituden sowie der Frequenzen der Meßgrößen zeitlich einzeln oder in funktionaler Abhängigkeit zur Erkennung von Neigungs- und Bewegungszustandsänderungen der gesundheitsgefährdeten Person genutzt wird.

Zur Durchführung des Verfahrens ist in dem von einer gesundheitsgefährdeten Person zu tragenden Überwachungsgerät ein Neigungs- und Bewegungssensor angeordnet, zur Erfassung von Neigungs- und Bewegungszustandsänderungen, wobei diese Informationen in einer Auswerteeinheit des Überwachungsgerätes mit Meßwertfolgen von bekannten Bewegungsabläufen der Person verglichen werden und wobei auftretende Differenzen ermittelt und bei Überschreitung vorgegebener maximaler Differenzwerte ein Voralarm ausgelöst wird, der bei Nichtlöschung durch ein an dem Überwachungsgerät angeordnetes Betätigungselement mittels eines Alarmgebers sowohl ein optisches oder akustisches Signal als auch einen durch Funk übertragenen Notruf auslöst.

Bevorzugt ist vorgesehen, daß der Neigungs- und Bewegungssensor in dem Überwachungsgerät als optisches Meßsystem ausgebildet ist, wobei eine in einer doppelwandigen Glaslinse bewegliche und von einer Lichtquelle beleuchtete Kugel bei Neigungs- und Bewegungsänderungen der gesundheitsgefährdeten Person unterschiedliche Schatten auf eine LCD-Kamera projiziert und damit unterschiedliche Signalverläufe in der LCD-Kamera erzeugt.

Bevorzugt ist weiterhin vorgesehen, daß der Neigungs- und Bewegungssensor in dem Überwachungsgerät als elektromechanisches Meßsystem ausgebildet ist, wobei mittels einer federnd gelagerten Masse sowie in zwei Ebenen angeordneten Induktionsspulen Informationen vom zeitlichen Verlauf von Neigungs- und Bewegungsänderungen der gesundheitsgefährdeten Person ermittelt werden.

Vorteilhaft ist es, daß der Neigungs- und Bewegungssensor einerseits zur Informationsgewinnung der Bewegungsabläufe der gesundheitsgefährdeten Person vorgesehen ist und andererseits die Bewegungsenergie in elektrische Energie umwandelt, so daß der vorhandene Energiespeicher des Überwachungsgerätes ständig aufgeladen ist.

Vorteilhaft ist weiterhin vorgesehen, daß die Lichtquelle des optischen Neigungs- und Bewegungssensors gleichzeitig als Kontrollampe für die Funktionsfähigkeit des am Oberkörper der gesundheitsgefährdeten Person zu tragenden Überwachungsgeräts vorgesehen ist.

Desweiteren ist bevorzugt vorgesehen, daß bei Anwendung eines Dauermagneten als Masseelement für den elektromechanischen Neigungs-und Bewegungssensor des Überwachungsgerätes die in seinen Spulen erzeugte Spannung gleichzeitig zur Energieerzeugungfür das Überwachungsgerät verwendet wird.

Eine vorteilhafte Ausführungsform wird darin gesehen, daß das Betätigungselement zur Löschung des Voralarms als Taste an dem Gehäuse des Überwachungsgerätes vorgesehen ist.

Eine weitere vorteilhafte Ausführungsform wird darin gesehen, daß der willensunabhängige Notruf mittels des in dem Überwachungsgerät angeordneten Alarmgebers erfolgt.

Auch kann vorteilhaft vorgesehen sein, daß durch manuelle Betätigung eines an dem Gehäuse des Überwachungsgerätes angeordneten Schalters ein willensabhängiger Notruf ausgelöst wird.

Vorteilhaft ist zudem, daß die einzelnen Bauelemente des Überwachungsgerätes sowohl in einem Gerät als auch in zwei durch Funk miteinander verbundenen Geräten vorgesehen sind.

Eine weitere bevorzugte Ausführungsform wird darin gesehen, daß die einzelnen Bauelemente durch Schachtelbauweise in dem Überwachungsgerät angeordnet sind.

Durch die Erfindung wird ein Verfahren und eine Anordnung zur Durchführung des Verfahrens zur Verfügung gestellt, mit denen lebensbedrohliche Situationen, wie eine Sturzsituation einer gesundheitsgefährdeten Person mittels eines am Körper der Person zu tragenden Überwachungsgerätes sicher und schnell erfaßt, bewertet sowie gegebenenfalls ein willensabhängiger und/oder willensunabhängige Notruf ausgelöst werden kann.

Weiterhin kann mit diesem Überwachungsgerät eine eindeutige Zuordnung von zeitlichen Bewegungsabläufen einer gesundheitsgefährdeten Person erfaßt werden, auch in einem größeren unübersichtlichen Gelände.

Ein Ausführungsbeispiel der Erfindung ist in der Zeichnung dargestellt und im folgenden näher beschrieben.
Es zeigt:
- Figur 1: eine Anordnung eines Überwachungsgerätes an einer Person
- Figur 2: ein Ausführungsbeispiel eines Überwachungsgerätes mit einem optischen Neigungs- und Bewegungssensor
- Figur 3: ein Ausführungsbeispiel eines Überwachungsgerätes mit einem optischen Neigungs- und Bewegungssensor in geneigter Lage
- Figur 4: ein Ausführungsbeispiel eines Überwachungsgerätes mit einem elektromechanischen Neigungs- und Bewegungssensor
- Figur 5: eine Draufsicht des elektromechanischen Neigungs- und Bewegungssensors
- Figur 6: eine Draufsicht des elektromechanischen Neigungs- und Bewegungssensors in geneigter Lage

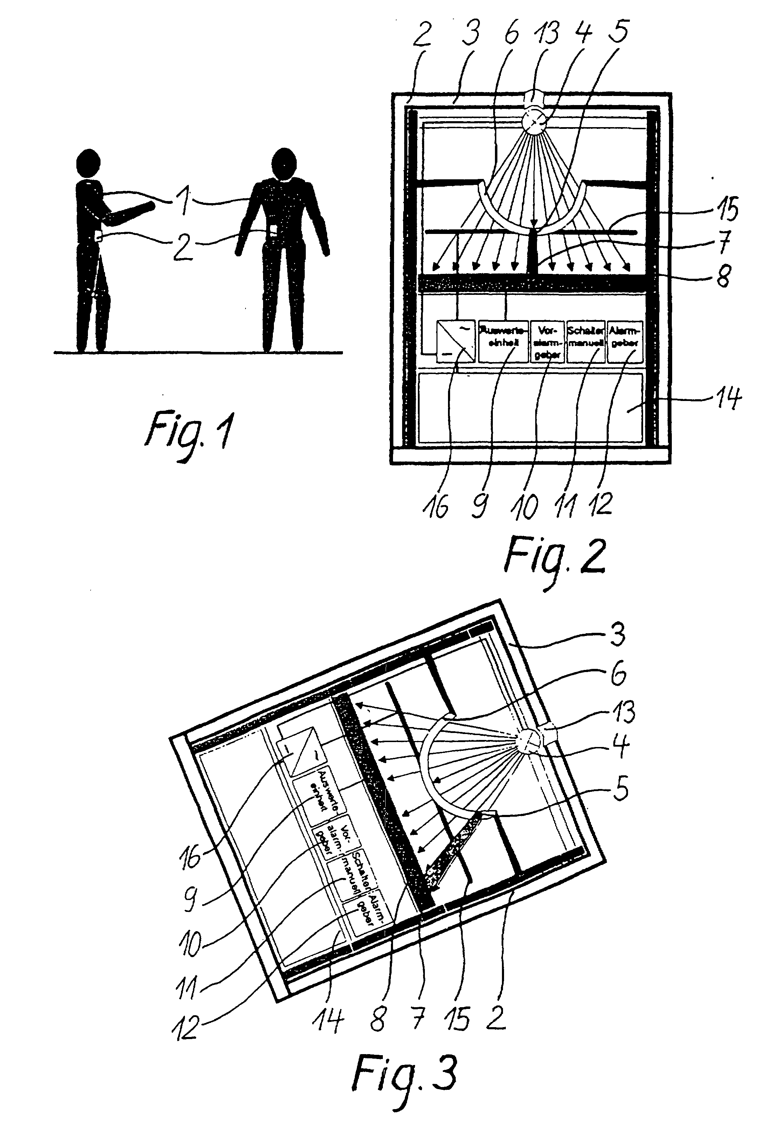

Mit dem Verfahren und der Anordnung zur Erfassung von Sturzsituationen gesundheitsgefährdeter Personen werden Neigungs- und Bewegungszustandsänderungen einer Person, sowie normale Bewegungen nach Schnelligkeit und Intensität vor einem Sturz, kurzzeitige gewollte oder ungewollte Lageänderungen einer Person, die Schnelligkeit einer Sturzsituation, eine Neigung, eine Bewegungstätigkeit und eine Bewegungsstärke nach einem Sturz und ein Zustand der Bewegungslosigkeit mittels eines entsprechend Figur 1 am Oberkörper 1 der gesundheitsgefährdeten Person angebrachten Überwachungsgerätes 2 erfaßt. Die Auswertung dieser dynamischen Kenngrößen erfolgt nach einem funktionellen Zusammenhang, wobei die Bewegungspositionen einer zu überwachenden Person sowie ihre charakteristischen Neigungsänderungen in Abhängigkeit von der Schwerkraft mit einem Neigungs- und Bewegungssensor des Überwachungsgerätes 2 gemessen werden. Dabei werden die Lage und der zeitliche Verlauf der mit dem Überwachungsgerät 2 gemessenen Schwingungen kritischen Neigungs- und Lageänderungen des Körpers einer gesundheitsgefährdeten Person zugeordnet, und die Schwingungen und deren zeitlicher Verlauf charakterisieren dann den Bewegungszustand einer gesundheitsgefährdeten Person. Die erhaltenen Meßgrößen und ihr zeitlicher Verlauf dienen zur Bildung und Abspeicherung von Verhaltensmustern in einem neuronalen Netz. Durch den Vergleich von Mustern vor, während und nach einer Sturzsituation einer gesundheitsgefährdeten Person wird daraus eine logische Entscheidung abgeleitet, die einen Notruf aktiviert. Die Auswertung der erhaltenen Meßgrößen erfolgt durch ihren funktionellen Zusammenhang untereinander, indem aus mindestens einer Meßgröße zwei funktionelle Abhängigkeiten und/oder aus drei zusätzlichen funktionellen Abhängigkeiten in einem neuronalem Netz Muster für gefährliche oder nicht gefährliche Sturzsituationen gebildet und gespeichert werden. Diese funktionellen Abhängigkeiten dienen dazu, daß unterschieden werden kann, ob eine gefährdete Person in ihrem Umfeld liegt, eine geneigte Haltung einnimmt oder aufrecht steht. Lebensbedrohliche Situationen werden beispielsweise durch einen Neigungsgrad des Oberkörpers einer Person und ihre Änderung in einem bestimmten Zeitintervall erfaßt, bei Festlegung vorgegebener Grenzwerte.

Zur Erfassung von Sturzsituationen ist, wie in Figur 2 dargestellt, in einem Gehäuse 3 des Überwachungsgerätes 2 eine Lichtquelle 4 angeordnet, die eine Kugel 5 beleuchtet, die sich in einer gewölbten doppelwandigen Glaslinse 6 bei Neigungs- und Bewegungsänderungen des Oberkörpers 1 der gesundheitsgefährdeten Person bewegt. Die Kugel 5 projiziert dabei einen Schatten 7 auf eine LCD-Kamera 8, der je nach Lage der Kugel 5 in der Glaslinse 6 bei Neigung des gesamten Überwachungsgerätes 2 entsprechend Figur 3 unterschiedliche Lagen auf der LCD-Kamera 8 einnimmt und so in Abhängigkeit der Bewegungsabläufe der gesundheitsgefährdeten Person unterschiedliche Signalverläufe in der LCD-Kamera 8 erzeugt, die in einer Auswerteeinheit 9 mit gespeicherten bekannten Bewegungsabläufen einer Person verglichen werden und bei Überschreitung vorgegebener Differenzwerte einen Sturz signalisieren. Aus den Informationen ist zusätzlich ableitbar, in welchem zeitlichen Verlauf ein Sturz erfolgte und wieweit sich die gesundheitsgefährdete Person nach dem Sturz noch bewegt. Ändert die betreffende Person ihre aufrechte Lage, wird durch die damit verbundene Änderung der Lage des Überwachungsgerätes 2 mittels eines Voralarmgebers 10 ein Voralarm, insbesondere durch ein akustisches oder optisches Signal ausgelöst. Wird dieser Voralarm von der betreffenden Person nach einem vorgegebenen Zeitintervall durch einen an dem Gehäuse 3 angeordneten Schalter oder einer Taste 11 nicht unterbrochen, wird durch einen Alarmgeber 12 ein Hauptalarm ausgelöst und durch insbesondere ein Funksignal ein Notruf ausgelöst.

Die in dem Gehäuse 3 angeordnete Lichtquelle 4 wird dabei gleichzeitig durch ein im Gehäuse 3 angeordnetes Sichtfenster 13 als Kontrolleuchte für die Bereitschaft des Überwachungsgerätes 2 genutzt. Die für die Lichtquelle 4, die LCD-Kamera 8, die Auswerteeinrichtung 9 und den Voralarmgeber 10 notwendige Energie wird einem Energiespeicher 14 entnommen. Durch eine magnetische Polarisation der Kugel 5 und durch die ständige Bewegung der Kugel 5 in der doppelwandigen Glaslinse 6 bei Bewegung und der Lageänderung der zu überwachenden Person wird in einer unterhalb der Glaslinse 6 angeordneten Spule 15 ständig elektrische Spannung induziert, die mittels eines Gleichrichters 16 gleichgerichtet und dem Energiespeicher 14 zugeführt wird.

Die einzelnen Bauelemente des Überwachungsgerätes 2 sind vorzugsweise durch eine Schachtelbauweise in dem Gehäuse 3 befestigt.

Figur 3 zeigt ein Ausführungsbeispiel eines Überwachungsgerätes 2 mit einem optischen Neigungs- und Bewegungssensor in geneigter Lage, wobei die Lageänderung der Kugel 5 in der Glaslinse 6 und die damit verbundene veränderte Schattenbildung auf der LCD-Kamera 8 zu erkennen ist.

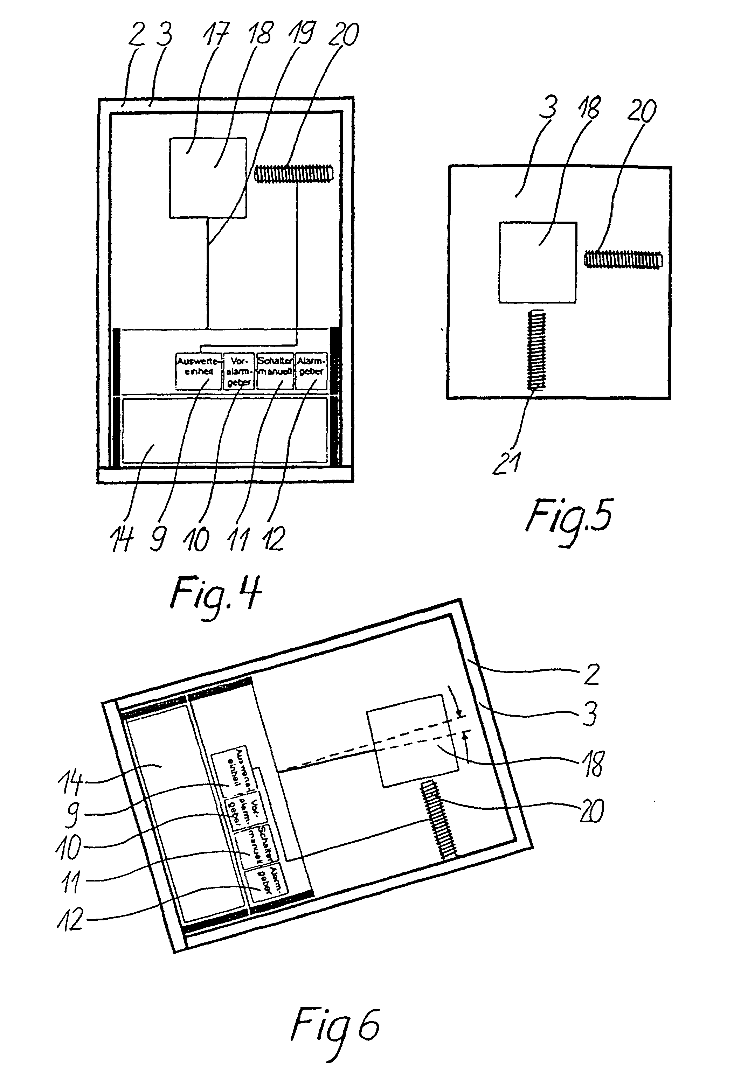

Figur 4 zeigt ein weiteres Ausführungsbeispiel für ein Überwachungsgerät 2, mit einem Neigungs- und Bewegungssensor, der eine federnd gelagerte Masse 17 enthält, welche aus einem Massenteil 18, einer Biegefeder 19 und entsprechend Figur 5 aus zwei im rechten Winkel zueinander angeordneten Spulen 20 und 21 besteht, wobei in Abhängigkeit von dem Abstand des Masseteilchens 18 von den Spulen 20 und 21 eine Induktivitätsänderung in den Spulen 20 und 21 erzeugt wird, welche zu einer Meßwertverarbeitung genutzt wird, wobei die Auswertung der Meßdaten nach demselben Verfahren wie bei dem Überwachungsgerät 2 mit einem optischen Neigungs- und Bewegungssensor mittels einer Kugel 5, wie in Figur 1 darge-stellt, erfolgt. Durch Verwendung eines Dauermagneten als Masseteilchen 18 kann neben der Meßwerterfassung auch Energie durch Induktion in den Spulen 20 und 21 erzeugt werden.

Figur 5 und 6 zeigen eine Draufsicht des elektromechanischen Neigungs-und Bewegungssensors in nicht geneigter und in geneigter Lage, wobei die Meßwerterfassung vorzugsweise mit zwei rechtwinklig zueinander angeordneten Spulen 20 und 21 durchgeführt wird.

Die Erfindung ist nicht auf das Ausführungsbeispiel beschränkt, sondern durch die Patentansprüche.

## Patentansprüche

1. Verfahren zur Erfassung von Sturzsituationen gesundheitsgefährdeter Personen, insbesondere Kranken, alten Menschen und Personen gefährdeter Berufsgruppen, wobei mittels eines am Körper der zu überwachenden Person zu tragenden Überwachungsgeräts (2) Informationen von zeitlichen Bewegungsabläufen und Bewegungszuständen vor, während sowie nach einer Sturzsituation einer gesundheitsgefährdeten Person er faßt sowie ausgewertet werden und bei auftretenden Differenzen ein Notruf ausgelöst wird, **dadurch gekennzeichnet,**
**daß** mittels des von der gesundheitsgefährdeten Person am der Oberkörper (1) zu tragenden Überwachungsgerätes (2) in vorgegebenen, wählbaren Zeitintervallen sowohl lage- und bewegungsabhängige Meßwertfolgen von Neigungs- und Lageänderungen der Person als auch Meßwertfolgen über die Geschwindigkeit sowie die Beschleunigung von Bewegungsabläufe und den Aufprall d Person mittels eines in dem Überwachungsgerät (2) vorgesehenen Neigungs-und Bewegungssensors erfaßt und mit Meßwertfolgen von bekannten vorgegebenen Bewegungsabbläufen der Person verglichen werden, wobei bei Überschreitung maximaler Differenzwerte als Folge einer Sturzsituation der gesundheitsgefährdeten Person ein Voralarm sowie ein willensabhängiger und/oder willensunabhängiger Notruf ausgelöst wird.

2. Verfahren nach Anspruch 1, **dadurch gekennzeichnet, daß** das von der gesundheitsgefährdeten Person zu tragende Überwachungsgerät (2) die Lage- und Neigungsänderungen dieser Person sowie deren zeitlichen Verlauf mittels eines in 2 Ebenen wirkenden Neigungs- und Bewegungssensors mißt, wobei die Meßgrößen, wie Geschwindigkeit, Beschleunigung, Neigung und Richtung erfaßt und die kritischen Neigungs- und Bewegungszustandsänderungen der gesundheitsgefährdeten Person als Muster in einem neuronalen Netz ermittelt sowie bewertet werden, wobei gleichzeitig vorliegende Informationen einzeln oder in einem funktionellen Zusammenhang zur Bewertung dienen und daraus nach Vergleich mit den in dem Überwachungsgerät (2) gespeicherten Meßwertfolgen vorgegebener bekannter Bewegungsabläufe der Person bei auftretenden Differenzen sowie bei Überschreitung maximaler Differenzwerte Signale gebildet werden, die einen Voralarm auslösen, der bei Nichtlöschung einen Notruf auslöst.

3. Verfahren nach Anspruch 1 und 2, **dadurch gekennzeichnet, daß** die Änderung der Amplituden sowie der Frequenz der Meßgrößen zeitlich einzeln oder in funktionaler Abhängigkeit zur Erkennung von Neigungs- und Bewegungszustandsänderungen der gesundheitsgefährdeten Person genutzt wird.

4. Anordnung zur Durchführung des Verfahrens nach einem der Ansprüche 1 bis 3, **dadurch gekennzeichnet, daß** in dem von einer gesundheitsgefährdeten Person (1) zu tragenden Überwachungsgerät (2) ein Neigungs- und Bewegungssensor angeordnet ist, zur Erfassung von Neigungs- und Bewegungszustandsänderungen, wobei diese Informationen in einer in dem Überwachungsgerät (2) vorgesehenen Auswerteeinheit (9) mit Meßwertfolgen von bekannten Bewegungsabläufen der Person verglichen werden und wobei auftretende Differenzen ermittelt und bei Überschreitung vorgegebener maximaler Differenzwerte ein Voralarm mittels eines Voralarmgebers (10) ausgelöst wird, der nach Nichtlöschung durch ein an dem Überwachungsgerät (2) angeordnetes Betätigungselement (11 )mittels eines Alarmgebers (12) sowohl ein optisches oder akustisches Signal als auch einen durch Funk übertragenen Notruf auslöst.

5. Anordnung zur Erfassung von Sturzsituationen gesundheitsgefährdeter Personen nach Anspruch 4, **dadurch gekennzeichnet, daß** der Neigungs- und Bewegungssensor in dem Überwachungsgerät (2) als optisches Meßsystem ausgebildet ist, wobei eine in einer doppelwandigen Glaslinse (6) bewegliche und von einer Lichtquelle (4) beleuchtete Kugel (5) bei Neigungs- und Bewegungsänderungen der gesundheitsgefährdeten Person (1) unterschiedliche Schatten (7) auf eine LCD-Kamera (6)projiziert und damit unterschiedliche Signalverläufe in der LCD-Kamera (8) erzeugt.

6. Anordnung zur Erfassung von Sturzsituationen gesundheitsgefährdeter Personen nach Anspruch 4 **dadurch gekennzeichnet, daß** der Neigungs- und Bewegungssensor in dem Überwachungsgerät (2) als elektromechanisches Meßsystem ausgebildet ist, wobei mittels einer federnd gelagerten Masse (17) sowie in zwei Ebenen angeordneten Induktionsspulen (20 und 21) Informationen vom zeitlichen Verlauf von Neigungs- und Bewegungsänderungen der gesundheitsgefährdeten Person (1) ermittelt werden.

7. Anordnung zur Erfassung von Sturzsituationen gesundheitsgefährdeter Personen nach einem der Ansprüche 4 und 6, **dadurch gekennzeichnet, daß** der Neigungs- und Bewegungssensor einerseits zur Informationsgewinnung der Bewegungsverläufe der gesundheitsgefährdeten Person (1) vorgesehen ist und anderseits die Bewegungsenergie in elektrische Energie umwandelt, so daß der vorhandene Energiespeicher (14) des Überwachungsgerätes (2) ständig aufgeladen wird.

8. Anordnung zur Erfassung von Sturzsituationen gesundheitsgefährdeter Personen nach Anspruch 5, **dadurch gekennzeichnet, daß** die Lichtquelle (4) des optischen Neigungs- und Bewegungssensors des Überwachungsgerätes (2) gleichzeitig als KontrolLlampe für die Funktionsfähigkeit des am Oberkörper (1) der gesundheitsgefährdeten Person zu tragenden Überwachungsgeräts (2) verwendet wird.

9. Anordnung zur Erfassung von Sturzsituationen gesundheitsgefährdeter Personen nach Anspruch 6, **dadurch gekennzeichnet, daß** bei Anwendung eines Dauermagneten als Masseelement (17) für den elektromechanischen Neigungs- und Bewegungssensor des Überwachungsgerätes (2) die in seinen Spulen (20 und 21) erzeugte Spannung gleichzeitig zur Energieerzeugung für das Überwachungsgerät (2) verwendet wird.

10. Anordnung zur Erfassung von Sturzsituationen gesundheitsgefährdeter Personen nach einem der Ansprüche 4 bis 9, **dadurch gekennzeichnet, daß** das Betätigungselement (11) zur Löschung des Voralarms als Taste an einem Gehäuse (3) des Überwachungsgerätes (2) vorgesehen ist.

11. Anordnung zur Erfassung von Sturzsituationen gesundheitsgefährdeter Personen nach einem der Ansprüche 4 bis 10, **dadurch gekennzeichnet, daß** ein willensunabhängiger Notruf mittels des in dem Überwachungsgerät (2) angeordneten Alarmgebers (12) erfolgt.

12. Anordnung zur Erfassung von Sturzsituationen gesundheitsgefährdeter Personen nach einem der Ansprüche 4 bis 11, **dadurch gekennzeichnet, daß** durch manuelle Betätigung eines Schalters (11) ein willensabhängiger Notruf ausgelöst wird.

13. Anordnung zur Erfassung von Sturzsituationen gesundheitsgefährdeter Personen nach einem der Ansprüche 4 bis 12, **dadurch gekennzeichnet, daß** die einzelnen Bauelemente des Überwachungsgerätes (2) sowohl in einem Gerät als auch in zwei durch Funk miteinander verbundenen Geräten vorgesehen sind.

14. Anordnung zur Erfassung von Sturzsituationen gesundheitsgefährdeter Personen nach einem der Ansprüche 4 bis 13, **dadurch gekennzeichnet, daß** die einzelnen Bauelemente durch Schachtelbauweise in dem Überwachungsgerät (2) angeordnet sind.

## Claims

1. Method of detecting fall situations of persons with a risk to health, particularly patients, elderly people and persons from occupational groups exposed to risk, wherein by means of a monitoring instrument (2), which is to be worn on the body of the person to be monitored, data relating to movement courses and movement states over time are detected and evaluated before, during and after a fall situation of a person with a risk to health and an emergency call is triggered in the case of arising differences, **characterised in that** by means of the monitoring instrument (2), which is to be worn at the upper body (1) by the person with a risk to health, not only position-dependent and movement-dependent measurement value sequences of changes in inclination and position of the person, but also measurement value sequences with respect to the speed as well as the acceleration of movement courses and the impact of the person are detected at predetermined, selectable time intervals by means of an inclination and movement sensor provided in the monitoring instrument (2) and are compared with measurement value sequences of known predetermined movement courses of the person, wherein in the case of exceeding of a maximum difference value as a consequence of a fall situation of the person with a risk to health a preliminary alarm as well as a voluntary and/or an involuntary emergency call is triggered.

2. Method according to claim 1, **characterised in that** the monitoring instrument (2), which is to be worn by the person with a risk to health, measures the changes in position and inclination of this person as well as the course in time thereof by means of an inclination and movement sensor acting in two planes, wherein the measurement values, such as speed, acceleration, inclination and direction, are detected and the critical changes in state of inclination and state of movement of the person with a risk to health are ascertained and assessed as a pattern in a neuronal network, wherein data which is present at the same time serve individually or in a functional relationship for assessment and signals, which trigger a preliminary alarm which triggers an emergency call if not cancelled, are formed therefrom after comparison with the measurement value sequences, which are stored in the monitoring instrument (2), of predetermined known movement courses of the person in the case of arising differences and in the case of exceeding maximum difference values.

3. Method according to claim 1 and 2, **characterised in that** the change in the amplitudes as well as the frequency of the measurement magnitudes over time is used individually or in functional dependence for recognition of changes in state of inclination and state of movement of the person with a risk to health.

4. Arrangement for carrying out the method according to one of claims 1 to 3, **characterised in that** in the monitoring instrument (2), which is to be worn by a person (1) with a risk to health, there is arranged an inclination and movement sensor for detection of changes in state of inclination and state of movement, wherein these data are compared in an evaluating unit (9), which is provided in the monitoring instrument (2), with measurement value sequences of known movement courses of the person and wherein arising differences are ascertained and in the case of exceeding of a predetermined maximum difference value a preliminary alarm is triggered by means of a preliminary alarm transmitter (10), which, after non-cancellation by an actuating element (11) arranged at the monitoring instrument (2), triggers by means of an alarm transmitter (12) not only an optical or acoustic signal, but also an emergency call transmitted by radio.

5. Arrangement for detection of fall situations of persons with a risk to health according to claim 4, **characterised in that** the inclination and movement sensor in the monitoring instrument (2) is constructed as an optical measuring system, wherein a ball (5), which is movable in a double-walled glass lens (6) and eliminated by a light source (4), projects different shadows (7) on an LCD camera (6) in the case of changes in inclination and movement of the person with a risk to health and thus generates different signal courses in the LCD camera (8).

6. Arrangement for detection of fall situations of persons with a risk to health according to claim 4, **characterised in that** the inclination and movement sensor in the monitoring instrument (2) is constructed as an electromechanical measuring system, wherein data with respect to the course over time of changes in inclination and movement of the person (1) with a health risk are detected by means of a resiliently mounted mass (17) as well as induction coils (20 and 21) arranged in two planes.

7. Arrangement for detection of fall situations of persons with a risk to health according to one of claims 4 and 6, **characterised in that** the inclination and movement sensor on the one hand is provided for obtaining data on the movement courses of the person (1) with a health risk and on the other hand convert the movement energy into electrical energy so that the energy store (14), which is present, of the monitoring instrument (2) is constantly charged.

8. Arrangement for detection of fall. situations of persons with a risk to health according to claim 5, **characterised in that** the light source (4) of the optical inclination and movement sensor of the monitoring instrument (2) is employed at the same time as a check lamp for the functional capability of the monitoring instrument (2) to be worn at the upper body (1) of the person with a risk to health.

9. Arrangement for detection of fall situations of persons with a risk to health according to claim 6, **characterised in that** in the case of use of a permanent magnet as a mass element (17) for the electromechanical inclination and movement sensor of the monitoring instrument (2) the voltage generated in the coils (20 and 21) thereof is at the same time used for generation of energy for the monitoring instrument (2).

10. Arrangement for detection of fall situations of persons with a risk to health according to one of claims 4 to 9, **characterised in that** the actuating element (11) for cancellation of the preliminary alarm is provided as a button at a housing (3) of the monitoring instrument (2).

11. Arrangement for detection of fall situations of persons with a risk to health according to one of claims 4 to 10, **characterised in that** an involuntary emergency call is effected by means of the alarm transmitter (12) arranged in the monitoring instrument (2).

12. Arrangement for detection of fall situations of persons with a risk to health according to one of claims 4 to 11, **characterised in that** a voluntary emergency call is triggered by manual actuation of a switch (11).

13. Arrangement for detection of fall situations of persons with a risk to health according to one of claims 4 to 12, **characterised in that** the individual components of the monitoring instrument (2) are provided not only in one device, but also in two devices connected together by radio.

14. Arrangement for detection of fall situations of persons with a risk to health according to one of claims 4 to 13, **characterised in that** the individual components are arranged in the monitoring instrument (2) by interlocking construction.

## Revendications

1. Procédé pour détecter la chute de personnes exposées à un risque sanitaire, en particulier de personnes malades, âgées et exerçant des professions dangereuses, dans lequel un appareil de surveillance à porter par la personne à surveiller saisit et évalue des informations de successions de mouvements dans le temps et d'états de mouvements avant, pendant et après la chute d'une personne exposée à un risque sanitaire et déclenche un appel de détresse en cas de différences, **caractérisé par le fait**
**que** l'appareil de surveillance (2) que la personne exposée à un risque sanitaire porte sur le torse (1) saisit à intervalles de temps définis, réglables, aussi bien des suites de valeurs mesurées de changements d'inclinaison et de position en fonction de la position et des mouvements de la personne que des suites de valeurs mesurées sur la vitesse ainsi que l'accélération de successions de mouvements et la chute de la personne au moyen d'un capteur d'inclinaison et de mouvement prévu dans l'appareil de surveillance (2) et les compare avec des suites de valeurs mesurées de successions de mouvements définies et connues de la personne, une préalarme ainsi qu'un appel de détresse dépendant et/ou indépendant de la volonté étant déclenchés en cas de dépassement de valeurs de différence maximales résultant d'une chute de la personne exposée à un risque sanitaire.

2. Procédé selon la revendication 1, **caractérisé par le fait que** l'appareil de surveillance (2) à porter par la personne exposée à un risque sanitaire mesure les changements d'inclinaison et de position de cette personne ainsi que leur déroulement dans le temps au moyen d'un capteur d'inclinaison et de mouvement opérant dans deux plans, dans lequel les grandeurs mesurées telles que vitesse, accélération, inclinaison et sens sont saisies et les changements critiques d'état d'inclinaison et de mouvement de la personne exposée à un risque sanitaire sont déterminés et évalués en tant que modèle dans un réseau neuronal, dans lequel en même temps des informations existantes sont utilisées individuellement ou en relation fonctionnelle pour former, après comparaison avec les suites de valeurs mesurées de successions de mouvements définies et connues de la personne enregistrées dans l'appareil de surveillance (2), des signaux qui, en cas de différences ou de dépassement de valeurs de différence maximales, déclenchent une préalarme qui déclenche un appel de détresse en cas de non-extinction.

3. Procédé selon les revendications 1 et 2, **caractérisé par le fait que** le changement d'amplitude et de fréquence des grandeurs mesurées dans le temps est utilisé isolément ou en dépendance fonctionnelle pour détecter des changements d'état d'inclinaison et de mouvement de la personne exposée à un risque sanitaire.

4. Dispositif pour réaliser le procédé selon l'une des revendications 1 à 3, **caractérisé par le fait que** l'appareil de surveillance (2) à porter par une personne (1) exposée à un risque sanitaire contient un capteur d'inclinaison et de mouvement pour la saisie de changements d'état d'inclinaison et de mouvement; ces informations sont comparées dans une unité d'évaluation (9) prévue dans l'appareil de surveillance (2) avec des suites de valeurs mesurées de successions de mouvements de la personne, les différences se produisant sont calculées et, en cas de dépassement de valeurs de différence maximales définies, une préalarme est déclenchée au moyen d'un émetteur de préalarme (10) qui, en cas de non-extinction par un élément de commande (11) disposé sur l'appareil de surveillance (2), déclenche au moyen d'un émetteur d'alarme (12) aussi bien un signal optique ou acoustique qu'un appel de détresse transmis par radio.

5. Dispositif pour détecter la chute de personnes exposées à un risque sanitaire sclon la revendication 4, **caractérisé par le fait que** le capteur d'inclinaison et de mouvement de l'appareil de surveillance (2) est réalisé sous la forme d'un système de mesure optique dans lequel une bille (5) mobile dans une lentille de verre (6) et éclairée par une source lumineuse (4) projette des ombres différentes (7) sur une caméra LCD (8) et génère ainsi des signaux différents dans la caméra LCD (8) lors de changements d'inclinaison et de mouvement de la personne (1) exposée à un risque sanitaire.

6. Dispositif pour détecter la chute de personnes exposées à un risque sanitaire selon la revendication 4, **caractérisé par le fait que** le capteur d'inclinaison et de mouvement de l'appareil de surveillance (2) est réalisé sous la forme d'un système de mesure électromécanique dans lequel les informations sur le déroulement dans le temps des changements d'inclinaison et de mouvement de la personne (1) exposée à un risque sanitaire sont obtenues au moyen d'une masse (17) suspendue par ressorts ainsi que de bobines d'induction (20 et 21) disposées dans deux plans.

7. Dispositif pour détecter la chute de personnes exposées à un risque sanitaire selon l'une des revendications 4 et 6, **caractérisé par le fait que le** capteur d'inclinaison et de mouvement est prévu d'une part pour recueillir des informations sur le déroulement séquentiel des mouvements de la personne (1) exposée à un risque sanitaire et d'autre part pour convertir l'énergie des mouvements en énergie électrique de sorte que l'accumulateur d'énergie (14) de l'appareil de surveillance (2) est chargé en permanence.

8. Dispositif pour détecter la chute de personnes exposées à un risque sanitaire selon la revendication 5, **caractérisé par le fait que** la source lumineuse (4) du capteur d'inclinaison et de mouvement optique de l'appareil de surveillance (2) est utilisée en même temps comme lampe de contrôle de la capacité de fonctionner de l'appareil de surveillance (2) à porter sur le torse (1) de la personne exposée à un risque sanitaire.

9. Dispositif pour détecter la chute de personnes exposées à un risque sanitaire selon la revendication 6, **caractérisé par le fait que** dans le cas de l'utilisation d'un aimant permanent comme masse (17) pour le capteur d'inclinaison et de mouvement électromécanique de l'appareil de surveillance (2), la tension générée dans ses bobines (20 et 21) est utilisée en même temps pour produire de l'énergie pour l'appareil de surveillance (2).

10. Dispositif pour détecter la chute de personnes exposées à un risque sanitaire selon l'une des revendications 4 à 9, **caractérisé par le fait que** l'élément de commande (11) servant à éteindre la préalarme se présente sous la forme d'une touche sur un boîtier (3) de l'appareil de surveillance (2).

11. Dispositif pour détecter la chute de personnes exposées à un risque sanitaire selon l'une des revendications 4 à 10, **caractérisé par le fait qu'**un appel de détresse indépendant de la volonté s'effectue au moyen de l'émetteur d'alarme (12) disposé dans l'appareil de surveillance (2).

12. Dispositif pour détecter la chute de personnes exposées à un risque sanitaire selon l'une des revendications 4 à 11, **caractérisé par le fait qu'**un appel de détresse dépendant de la volonté est déclenché par commande manuelle d'un commutateur (11).

13. Dispositif pour détecter la chute de personnes exposées à un risque sanitaire selon l'une des revendications 4 à 12, **caractérisé par le fait que** les différents éléments de l'appareil de surveillance (2) sont prévus aussi bien dans un appareil que dans deux appareils reliés entre eux par radio.

14. Dispositif pour détecter la chute de personnes exposées à un risque sanitaire selon l'une des revendications 4 à 13, **caractérisé par le fait que** les différents éléments sont assemblés par emboîtement dans l'appareil de surveillance (2).
